# EUROPÄISCHE PATENTANMELDUNG

(11) **EP 2 279 705 A1**
(43) Veröffentlichungstag der Anmeldung: **02.02.2011**
(21) Anmeldenummer: 09166597.6
(22) Anmeldetag: 28.07.2009
(51) Int. Cl.: A61B 17/70

(54) **Wirbelsäulenimplantat**

(71) Anmelder: Spinelab AG, 6304 Zug (CH)
(72) Erfinder: Zehnder, Thomas, 8806 Bäch (CH); Braunschweiler, Reto, 8413 Neftenbach (CH)
(74) Vertreter: BOVARD AG

(57) **Zusammenfassung**

Ein Wirbelsäulenimplantat (1) umfasst Pedikelschrauben (2), die jeweils mit einem Kopfteil (5) und einem Einschraubteil (4) ausgestattet sind, welche in den jeweiligen Wirbelkörper einschraubbar sind. Diese Pedikelschrauben (2) sind über ein stabförmiges Verbindungselement (3) verbunden, das mindestens Teilbereiche aufweist, die aus einem elastischen Material gebildet sind. Die elastischen Bereiche sind formschlüssig mit den Kopfteilen (5) der Pedikelschrauben (2) beziehungsweise formschlüssig mit den Zwischenstücken verbindbar. Das stabförmige Verbindungselement (3) ist mit einer durchgehenden Längsbohrung (7) versehen, in welche ein zugund/oder druckstabiles Längselement (8) eingesetzt ist, das an beiden Endbereichen mit Anschlagelementen (9), (10) versehen ist, welche auf den stirnseitigen Endflächen des stabförmigen Verbindungselementes (3) abstützbar sind. Durch dieses erfindungsgemässe Wirbelsäulenimplantat kann eine Wirbelsäule in optimaler Weise abgestützt und stabilisiert werden.

## Beschreibung

Die vorliegende Erfindung bezieht sich auf ein Wirbelsäulenimplantat, umfassend Pedikelschrauben, die jeweils mit einem Kopfteil und einem Einschraubteil ausgestattet sind, welche mit den Einschraubteilen in die Wirbelkörper einschraubbar sind, mindestens ein stabförmiges Verbindungselement, das mindestens Teilbereiche aufweist, die aus einem elastischen Material gebildet sind, welche gegebenenfalls mit aus starrem Material bestehenden Zwischenstücken verbunden sind, mit welchem stabförmigem Verbindungselement die Pedikelschrauben miteinander verbindbar sind, welche elastischen Bereiche mindestens teilweise formschlüssig mit den Kopfteilen der Pedikelschrauben beziehungsweise mindestens teilweise formschlüssig mit den Zwischenstücken verbindbar sind.

Derartige Wirbelsäulenimplantate werden in Wirbelsäulen eines Patienten eingesetzt, um diese Wirbelsäule zu stabilisieren. Hierzu werden die Pedikelschrauben jeweils in einen Wirbelkörper der zu stabilisierenden Wirbelsäule eingeschraubt, in die Kopfteile der Pedikelschrauben wird das stabförmige Verbindungselement eingesetzt, der Kopfteil wird mit einer Verschlusseinrichtung verschlossen, das stabförmige Verbindungselement wird dadurch in der jeweiligen Pedikelschraube gehalten. Durch die Verwendung eines stabförmigen Verbindungselementes, das mindestens Teilbereiche aufweist, die aus einem elastischen Material gebildet sind, wird erreicht, dass die über elastische Zwischenstücke verbundenen Wirbelkörper der so stabilisierten Wirbelsäule nicht vollständig fixiert werden, wie dies beispielsweise bei der Verwendung eines Stabes aus Stahl auftreten würde, eine geringe Bewegung zwischen den einzelnen stabilisierten Wirbelkörpern ist möglich, wodurch vermieden wird, dass die Wirbel durch eine Verknöcherung sich miteinander verbinden.

Ein derartiges Wirbelsäulenimplantat ist beispielsweise aus der EP1364622 B1 bekannt. Mit diesem Wirbelsäulenimplantat wird eine erkrankte Wirbelsäule in optimaler Weise stabilisiert, der elastische Stab wird ebenfalls in optimaler Weise in den jeweiligen Pedikelschrauben gehalten, was durch die formflüssige Verbindung zwischen Stab und Pedikelschraube erreicht wird. Dieser Stab besteht vorteilhafterweise aus einem biokompatiblen Kunststoff auf Basis von Polyurethan, wodurch optimale Festigkeitseigenschaften mit vernünftigen geometrischen Abmessungen erreichbar sind.

Bei Patienten mit Spondylolisthesen sind derartige elastische Stäbe nicht optimal geeignet, da sie die Kräfte, die beim Wirbelgleiten nach vorne auftreten, nicht optimal aufnehmen können. Ebenfalls nicht optimal geeignet sind derartige Stäbe bei der sogenannten Hypermobilität einer Wirbelsäule. Der eingesetzte elastische Stab könnte beispielsweise überbelastet werden.

Die Aufgabe der vorliegenden Erfindung besteht somit darin, ein Wirbelsäulenimplantat zu schaffen, das einerseits eine Wirbelsäule in optimaler Weise stabilisiert, das aber andererseits auch die Kräfte aufnehmen kann, die, wie oben erwähnt, beispielsweise bei Spondylolisthesen oder Hypermobilität auftreten können.

Erfindungsgemäss erfolgt die Lösung dieser Aufgabe dadurch, dass das stabförmige Verbindungselement mit einer durchgehenden Längsbohrung versehen ist, in welche ein im wesentlichen zug- und/oder druckstabiles Längselement eingesetzt ist, das an beiden Endbereichen mit Anschlagelementen versehen ist, welche auf den stirnseitigen Endflächen des stabförmigen Verbindungselementes abstützbar sind.

Mit einem derartig ausgebildeten Wirbelsäulenimplantat lassen sich neben der optimalen stabilisierenden Unterstützung der Wirbelsäule mit den entsprechenden dämpfenden Eigenschaften auf Zug und Druck auch die translatorischen Kräfte, wie sie insbesondere bei Spondylolisthesen auftreten können, optimal aufnehmen.

In vorteilhafter Weise sind mindestens die Verbindungsstellen, mit welchen die elastischen Bereiche des stabförmigen Verbindungselementes mit den Kopfteilen der Pedikelschrauben beziehungsweise mit den Zwischenstücken verbindbar sind, mit strukturierten Oberflächen ausgestattet, welche mit entsprechend strukturierten Oberflächen der zu verbindenden Teile zusammenwirken, wodurch sich eine optimale formschlüssige Verbindung ergibt.

In vorteilhafter Weise bestehen die strukturierten Oberflächen aus Rippen und Rillen, die jeweils ineinander greifen, welche einfach herstellbar und montierbar sind.

In vorteilhafter Weise sind die Rippen und Rillen schraubenlinienförmig ausgebildet, wodurch sich entsprechende Verbindungselemente optimal zusammenfügen lassen.

In vorteilhafter Weise ist mindestens eines der beiden Anschlagelemente des Längselementes verstellbar ausgebildet, sodass sich eine maximale Zugdehnung des stabförmigen Verbindungselementes begrenzen lässt.

In vorteilhafter Weise ist das verstellbare Anschlagelement mit einem Innengewinde versehen, welches auf den mit einem entsprechenden Gewinde versehenen Endbereich des Längselementes aufschraubbar und sichtbar ist, wodurch eine stufenlose Verstellmöglichkeit erreicht wird.

In vorteilhafter Weise ist das verstellbare Anschlagelement mit einer Kappe versehen, welche den Endbereich des stabförmigen Verbindungselementes übergreift, wodurch ein optimaler Sitz erreicht wird.

Eine weitere vorteilhafte Ausgestaltung der Erfindung besteht darin, dass die Kappen des Anschlagelementes mit den aus elastischem Material bestehenden jeweiligen Endbereichen des stabförmigen Verbindungselementes im Wesentlichen formschlüssig verbunden sind. Dadurch wird erreicht, dass neben Zugkräften auch Druckkräfte auf das Längselement übertragen werden können, wodurch sowohl die Dämpfungseigenschaften als auch die Ermüdungseigenschaften (Kaltfluss) des stabförmigen Verbindungselementes optimiert werden können.

In vorteilhafter Weise ist mindestens eine der beiden Kappen zwischen zwei Stellelementen verschiebbar auf dem Längselement gehalten, was eine optimale Einstellung der zulässigen Bewegungsmöglichkeiten ergibt.

In vorteilhafter Weise sind auf die elastischen Bereiche des stabförmigen Verbindungselementes, wenn diese in die Kopfteile der Pedikelschrauben einzusetzen sind, Hülsen aufgesetzt, welche den jeweiligen elastischen Bereich des stabförmigen Verbindungselementes umschliessen, wodurch die Halterung und Montage dieser elastischen Bereiche in den Kopfteilen der Pedikelschrauben verbessert wird.

Um das Aufsetzen der Hülsen auf das stabförmige Verbindungselement zu vereinfachen, sind diese aus zwei Hälften gebildet, die auf der einen Längsseite gelenkig miteinander verbunden sind, während die gegenüber liegenden Längsseiten als Anschlagflächen dienen.

In vorteilhafter Weise weisen die Innenflächen der Hülsen eine strukturierte Oberfläche auf, welche der strukturierten Oberfläche des stabförmigen Verbindungselementes entspricht, wodurch auch hier eine im Wesentlichen formschlüssige Verbindung erhalten wird.

Ausführungsformen der Erfindung werden nachfolgend anhand der beiliegenden Zeichnungen beispielhaft näher erläutert.

Es zeigt,
Fig. 1 in räumlicher Darstellung eine erste Ausführungsform eines erfindungsgemässen Wirbelsäulenimplantats;
Fig. 2 eine Schnittdarstellung des Wirbelsäulenimplantats gemäss Fig. 1;
Fig. 3 eine schematische Schnittdarstellung des Wirbelsäulenimplantats mit drei Pedikelschrauben;
Fig. 4 eine Schnittdarstellung eines Wirbelsäulenimplantats, bei welchem das Längselement mit Anschlagelementen versehen ist, die formschlüssig mit dem stabförmigen Verbindungselement verbunden sind;
Fig. 5 in räumlicher Darstellung ein Wirbelsäulenimplantat, bei welchem das stabförmige Verbindungselement einen elastischen Teilbereich und starre Zwischenstücke aufweist;
Fig. 6 eine Schnittdarstellung durch das Wirbelsäulenimplantat gemäss Fig. 5;
Fig. 7 eine Schnittdarstellung durch ein Wirbelsäulenimplantat für drei Pedikelschrauben;
Fig. 8 eine Ansicht auf das Wirbelsäulenimplantat gemäss Fig. 7;
Fig. 9 eine Schnittdarstellung durch ein Wirbelsäulenimplantat, bei welchem auf die elastischen Teilbereiche im Bereich der Pedikelschrauben Hülsen aufgesetzt sind;
Fig. 10 eine Querschnittdarstellung des stabförmigen Verbindungselementes, mit aufgesetzter Hülse, das in den Kopfteil einer Pedikelschraube eingesetzt ist;
Fig. 11 eine Schnittdarstellung eines Wirbelsäulenimplantats mit drei Pedikelschrauben, mit auf das stabförmige Verbindungselement aufgesetzten Hülsen.

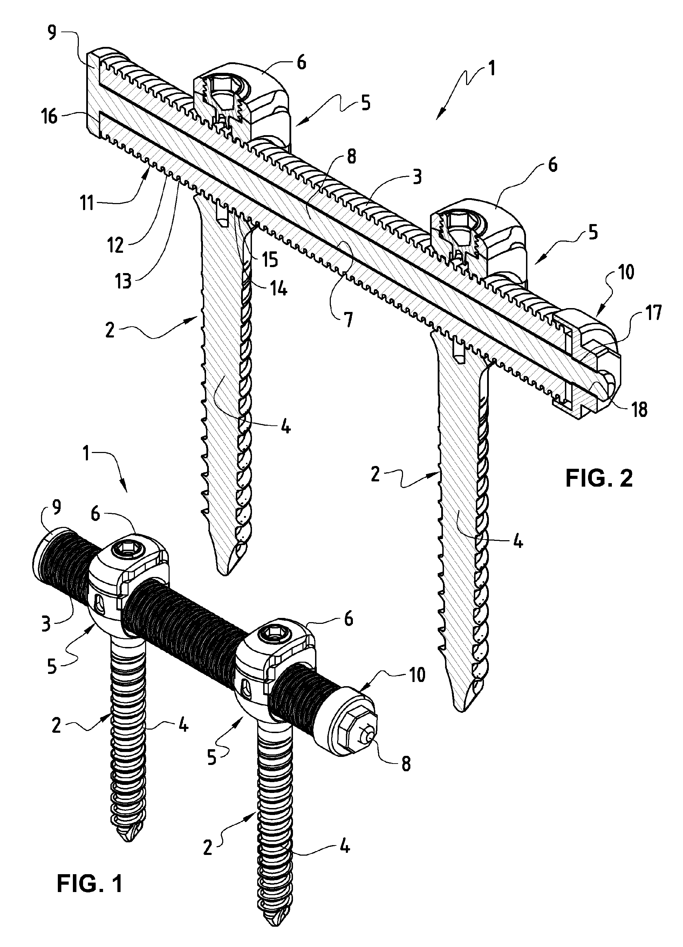

Fig. 1 zeigt ein Wirbelsäulenimplantat 1, das zwei Pedikelschrauben 2 und ein stabförmiges Verbindungselement 3 umfasst. Die Pedikelschrauben 2 werden mit ihrem Einschraubteil 4 jeweils in den entsprechenden Wirbelkörper eingeschraubt, das stabförmige Verbindungselement 3 wird in den jeweiligen Kopfteil 5 der Pedikelschraube 2 eingesetzt und durch eine Verschlusseinrichtung 6 in der Pedikelschraube 2 gehalten. Eine derartige Verschlusseinrichtung ist beispielsweise in der europäischen Patentanmeldung mit der Veröffentlichungsnummer EP 2074957 A1 beschrieben. Das in die Pedikelschrauben 2 eingesetzte stabförmige Verbindungselement besteht aus einem elastischen Material, beispielsweise aus einem biokompatiblen Kunststoff auf Basis von Polyurethan. In bekannter Weise wird eine Wirbelsäule, in welcher ein derartiges Wirbelsäulenimplantat eingesetzt ist, in optimaler Weise unterstützt, eine gewisse Bewegung zwischen den beiden Wirbel körpern wird zugelassen.

In eine zentral im stabförmigen Verbindungselement angebrachte, durchgehende Längsbohrung 7 (Fig. 2) ist ein Längselement 8 eingesetzt, dessen beide Endbereiche mit Anschlagelementen 9 und 10 ausgestattet sind.

Aus der Schnittdarstellung durch das Wirbelsäulenimplantat 1 gemäss Fig. 1 ist in Fig. 2 ersichtlich, wie das stabförmige Verbindungselement 3 in die Kopfteile 5 der Pedikelschrauben 2 eingesetzt und durch die Verschlusseinrichtungen 6 gehalten ist. Das stabförmige Verbindungselement 3 ist mit einer strukturierten Oberfläche 11 ausgestattet, die im hier dargestellten Ausführungsbeispiel aus Rippen 12 und Rillen 13 gebildet ist. Diese Rippen 12 und Rillen 13 greifen in entsprechende Rippen 14 und Rillen 15 ein, die in den entsprechenden Aufnahmebereichen am Kopfteil 5 der Pedikelschrauben 2 und den Verschlusseinrichtungen 6 angebracht sind. Dadurch wird zwischen den Pedikelschrauben 2 und dem stabförmigen Verbindungselement 3 jeweils eine formschlüssige Verbindung erhalten, wodurch die axial wirkenden Kräfte optimal übertragen werden können.

In die Längsbohrung 7, welche das stabförmige Verbindungselement 3 in axialer Richtung vollständig durchdringt, ist das Längselement 8 eingesetzt. Dieses Längselement 8 ist an den beiden Endbereichen jeweils mit einem Anschlagelement 9 bzw. 10 versehen. Im hier dargestellten Ausführungsbeispiel ist das eine Anschlagelement 9 fest mit dem Längselement 8 verbunden, es stützt sich auf der entsprechenden stirnseitigen Endfläche 16 des stabförmigen Verbindungselementes 3 ab.

Das gegenüberliegende Anschlagelement 10 ist verstellbar ausgebildet, es ist mit einem Innengewinde 17 versehen, mit welchem das Anschlagelement auf den mit einem entsprechenden Gewinde 18 ausgestatteten Endbereich des Längselementes 8 aufgeschraubt werden kann. In bekannter Weise kann dieses auf das Längselement 8 aufgeschraubte Anschlagelement 10 in der eingestellten Position gesichert werden, was beispielsweise durch Klebemittel oder in anderer bekannter Weise erfolgen kann.

Im in eine Wirbelsäule eingesetzten Zustand bewirkt dieses Wirbelsäulenimplantat 1, dass beim Beugen des menschlichen Oberkörpers nach hinten die beiden Kopfteile 5 der Pedikelschrauben 6 aufeinander zu bewegt werden. Dies bedeutet, dass der zwischen diesen beiden Pedikelschrauben 2 sich befindende Bereich des stabförmigen Verbindungselementes 3 zusammengepresst wird und eine entsprechende gedämpfte Bewegung der Wirbelsäule zugelassen wird. Beim Beugen der Wirbelsäule nach vorne bewegen sich die beiden Kopfteile der Pedikelschrauben 2 voneinander weg, der Bereich des stabförmigen Verbindungselementes 3 zwischen den beiden Pedikelschrauben 2 wird gedehnt, die jeweiligen über die Pedikelschrauben 2 vorstehenden Bereiche des stabförmigen Verbindungselementes 3 werden, da sie durch die Anschlagelemente 9 und 10 des Längselementes 8 gehalten werden, zusammengepresst, der Widerstand, der einer Beugungsbewegung der Wirbelsäule entgegengesetzt wird, nimmt ab dem Moment überproportional zu, in welchem die beiden über die Pedikelschrauben 2 vorstehenden Endbereiche des stabförmigen Verbindungselementes 3 an den Anschlagelementen 9 und 10 anstossen und komprimiert werden. Dadurch wird vermieden, dass das stabförmige Verbindungselement 3 zwischen den beiden Pedikelschrauben 2 übermässig auf Zug beansprucht wird, zudem wird vermieden, dass eine zu grosse Bewegung zugelassen wird, wobei die Endlagen dieser Bewegung in optimaler Weise gedämpft werden.

Mit der Verstellmöglichkeit des Anschlagelementes 10 kann die zulässige Bewegung der Wirbelsäule begrenzt werden, wobei die Bewegungsendlagen in optimaler Weise gedämpft werden, dies kann auch noch dadurch beeinflusst werden, dass die über die Pedikelschrauben 2 vorstehenden Bereiche des stabförmigen Verbindungselementes 3 und entsprechend das Längselement 8 hinsichtlich der Längenabmessungen ausgewählt werden können.

Das stabförmige Verbindungselement 3 und das Längselement 8 mit den Anschlagelementen 9 und 10 können aus entsprechenden einzelnen Bestandteilen zusammengesetzt und vormontiert werden, wodurch bei einem entsprechenden Operationsvorgang das fertig vormontierte stabförmige Verbindungselement mit eingesetztem und positioniertem Längselement in die in die Wirbelkörper eingeschraubten Pedikelschrauben eingesetzt und fixiert werden kann.

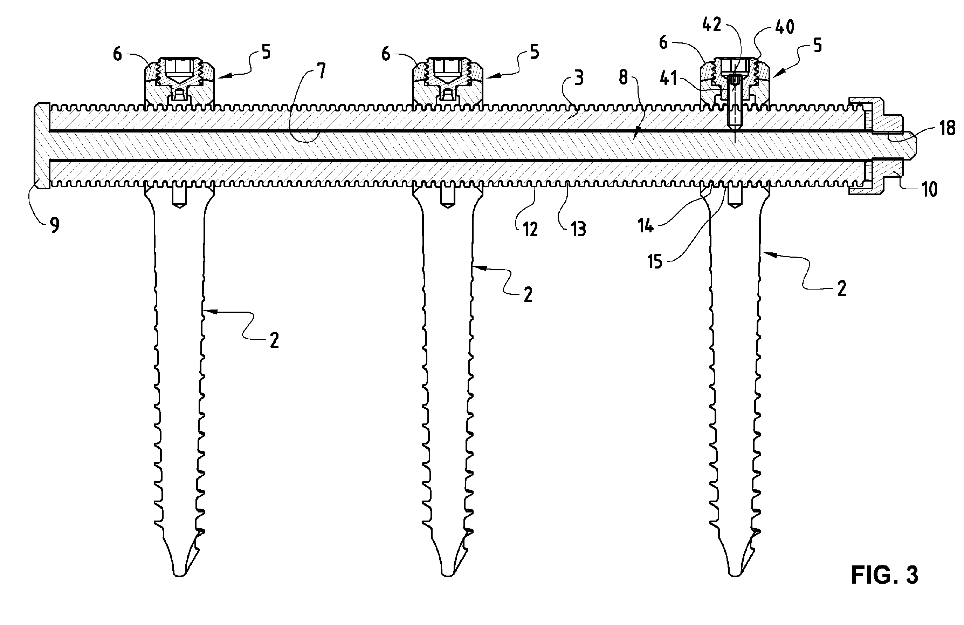

Fig. 3 zeigt ein Wirbelsäulenimplantat 1, das gemäss dem gleichen Prinzip aufgebaut ist, wie dasjenige, das in den Fig. 1 und 2 beschrieben worden ist. Beim hier vorliegenden Wirbelsäulenimplantat 1 werden drei Pedikelschrauben 2 mit dem einen stabförmigen Verbindungselement 3 verbunden, in die Längsbohrung 7 des stabförmigen Verbindungselementes 3 ist wiederum ein Längselement 8 eingesetzt, das beidseitig mit jeweils einem Anschlagelement 9 und 10 versehen ist, wobei das Anschlagelement 10 wiederum verstellbar auf dem Längselement 8 angeordnet ist. Das stabförmige Verbindungselement 3 ist wiederum mit Rippen 12 und Rillen 13 versehen, welche in entsprechende Rippen 14 und Rillen 15 des Kopfteils 5 der Pedikelschrauben 2 eingreifen und somit wiederum eine formschlüssige Verbindung entsteht.

Beim Beugen des Oberkörpers und demzufolge der Wirbelsäule nach hinten, in welcher dieses Wirbelsäulenimplantat 1 eingesetzt ist, werden die Bereiche des stabförmigen Verbindungselementes 3, die sich zwischen den Pedikelschrauben 2 befinden, zusammengepresst, wodurch die Wirbelkörper, in welcher die Pedikelschrauben 2 eingeschraubt sind, gestützt werden. Beim Beugen der Wirbelsäule nach vorne, in welche dieses Wirbelsäulenimplantat 1 eingesetzt ist, werden die Bereiche des stabförmigen Verbindungselementes 3, die sich zwischen den Pedikelschrauben 2 befinden, gedehnt, die über die beiden äusseren Pedikelschrauben 2 vorstehenden Bereiche des stabförmigen Verbindungselementes werden durch das Längselement 8 mit den daran angefügten Anschlagelementen 9 und 10 zusammengepresst, wodurch die Endlage des Beugens der Wirbelsäule wiederum entsprechend dem Wirbelsäulenimplantat gemäss Fig. 1 und Fig. 2 gedämpft wird. Selbstverständlich wird eine entsprechende Wirkungsweise auch bei einer grösseren Anzahl von Schrauben erreicht.

In Fig. 3 ist zusätzlich eine Ausführungsvariante für die Ausgestaltung des Kopfteils 5 einer Pedikelschraube 2 dargestellt. In der in Fig. 3 oben dargestellten Pedikelschraube 2 ist in der Spannschraube 40 des Kopfteils 5 ein Gewindeloch 41 angebracht, das axial angeordnet ist. Im dieses Gewindeloch 41 kann eine Madenschraube 42 eingeschraubt werden, welche das stabförmige Verbindungselement 3 durchdringen kann und das Längselement 8 bezüglich der Pedikelschraube 2 fixieren kann. Dadurch kann erreicht werden, dass diese Pedikelschraube 2 als Basis für die zulässige Bewegungsmöglichkeit des Längselementes 5 dienen kann. Es ist auch denkbar, dass durch Anbringen dieser Madenschrauben 42 im Kopfteil 5 der Pedikelschrauben 2 ein Wirbelsäulenimplantat, das zur Stabilisierung der einzelnen Wirbelkörper untereinander eingesetzt wurde, das gewünschte Resultat nicht erbringt, und die Wirbelkörper fixiert werden müssen. Mit dieser Möglichkeit muss das in die Wirbelsäule eingesetzte Wirbelsäulenimplantat nicht ersetzt werden, vielmehr kann in einem kleinen operativen Eingriff durch Einsetzen der Madenschrauben 42 eine nachträgliche Fixierung erreicht werden.

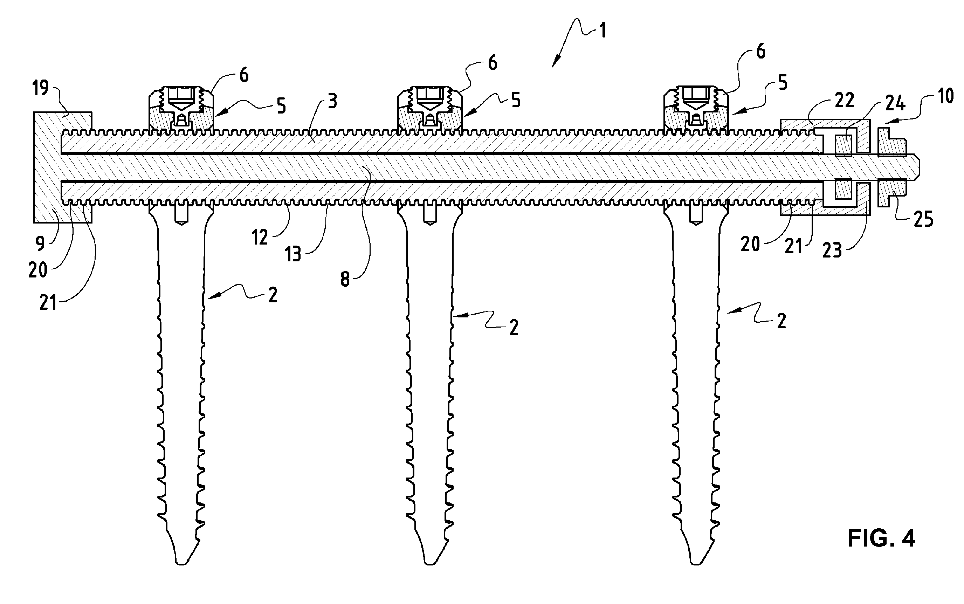

Das Wirbelsäulenimplantat 1, wie es in Fig. 4 dargestellt ist, ist gleich aufgebaut, wie das Wirbelsäulenimplantat 1 gemäss der Fig. 3, wobei aber die Anschlagelemente 9 und 10 des Längselementes 8 anders ausgebildet sind. Das Anschlagelement 9, das mit dem Längselement 8 fix verbunden ist, weist eine Kappe 19 auf, die den Endbereich des stabförmigen Verbindungselementes 3 übergreift. Der übergreifende Bereich der Kappe 19 weist ebenfalls Rippen 20 und Rillen 21 auf, die in die Rippen 12 und Rillen 13 des stabförmigen Verbindungselementes 3 eingreifen und zwischen Längselement 8 und stabförmigem Verbindungselement 3 somit eine formschlüssige Verbindung erhalten wird. Das verstellbare Anschlagelement 10 umfasst ebenfalls eine Kappe 22, welche ihrerseits den Endbereich des stabförmigen Verbindungselementes 3 übergreift und ebenfalls über Rippen 20 und Rillen 21 mit diesem formschlüssig verbunden ist. Dieses Anschlagelement 10 ist wiederum verstellbar, die Kappe 22 umfasst einen Steg 23, der zwischen zwei schraubbaren Elementen 24 und 25, die auf das Gewinde 18 des Längselementes 8 aufschraubbar sind und in bekannter Weise mit diesem gesichert werden können. Durch diese beiden schraubbaren Elemente 24 und 25, die als Stellelemente dienen, kann der verschiebbare Bereich des Anschlagelementes 10 eingestellt werden.

Die Wirkungsweise dieses in Fig. 4 dargestellten Wirbelsäulenimplantates ist wie folgt:

Beim Beugen der Wirbelsäule nach vorne werden die zwischen den Pedikelschrauben 2 sich befindenden Bereiche des stabförmigen Verbindungselementes 3 gestreckt, die beiden über die Pedikelschrauben 2 beidseits vorstehenden Endbereiche des stabförmigen Verbindungselementes 3 werden entsprechend der Ausführungsform gemäss Fig. 3 zusammengedrückt, der Steg 23 der Kappe 22 stösst dabei am schraubbaren Element 25 an. Beim Beugen der Wirbelsäule nach hinten werden die zwischen den Pedikelschrauben sich befindenden Bereiche des stabförmigen Verbindungselementes 3 zusammengedrückt, zusätzlich werden die über die Pedikelschrauben 2 vorstehenden Endbereiche des stabförmigen Verbindungselementes 3 gedehnt, wobei der Steg 23 der Kappe 22 sich auf dem schraubbaren Element 24 abstützt. Dadurch wird zusätzlich eine Endlagendämpfung der Streckbewegung einer Wirbelsäule erreicht.

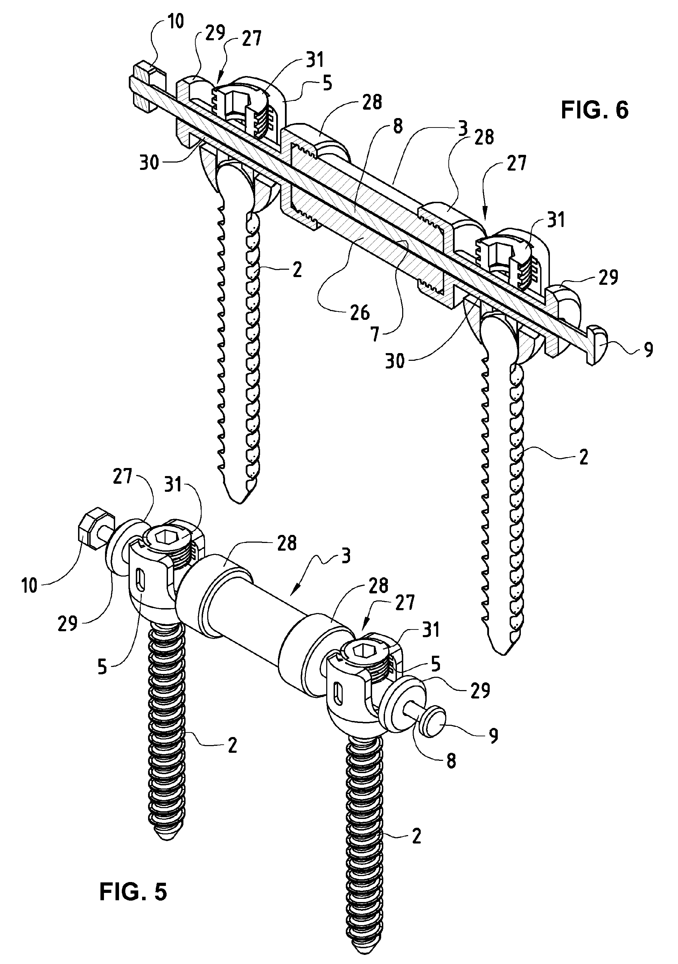

In den Fig. 5 und 6 ist ein weiteres Wirbelsäulenimplantat 1 dargestellt, bei welchem das stabförmige Verbindungselement 3 aus einem aus elastischem Material gefertigten Zwischenteil 26 besteht, der beidseitig mit einem hantelförmigen Element 27 aus starrem Material verbunden ist. Diese hantelförmigen Elemente 27 sind jeweils mit einer Kappe 28 ausgestattet, welche mit dem elastischen Zwischenteil 26 formschlüssig verbunden sind. Das jeweils der Kappe 28 abgewandte Ende des hantelförmigen Elements 27 ist mit einem Flansch 29 abgeschlossen. Zwischen Kappe 28 und Flansch 29 ist ein zylinderförmiger Bereich 30 vorgesehen, dieser zylinderförmige Bereich 30 wird jeweils in den Kopfteil 5 der Pedikelschrauben 2 eingesetzt und in bekannter Weise über Spannschrauben 31 in der Pedikelschraube festgeklemmt. Das stabförmige Verbindungselement 3 mit den beiden hantelförmigen Elementen 27 ist wiederum mit einer durchgehenden Längsbohrung 7 versehen, in welche das Längselement 8 eingesetzt ist, das wiederum mit einem fest mit dem Längselement 8 verbundenen Anschlagelement 9 und einem verstellbaren Anschlagelement 10 ausgestattet ist.

Die Funktionsweise dieses in den Fig. 5 und 6 dargestellten Wirbelsäulenimplantates 1 entspricht denjenigen, wie sie in den Fig. 1 und 2 dargestellt sind, beim Beugen der Wirbelsäule nach vorne wird das elastische Zwischenteil 26 des stabförmigen Verbindungselementes 3 gedehnt, wobei die Endlage durch die Anschlagelemente 9 und 10 des Längselementes 8 festgelegt wird, beim Beugen der Wirbelsäule nach hinten wird das elastische Zwischenteil 26 zusammengedrückt, wodurch wiederum die dämpfende Unterstützung der Wirbelsäule erreicht wird. Da der Querschnitt des zylinderförmigen Bereichs des stabförmigen Verbindungselementes 3 im Bereich des Pedikelschraubenkopfes geringer sein kann, als der Querschnitt des elastischen Zwischenstücks 26, können auch bekannte polyaxiale Pedikelschrauben verwendet werden.

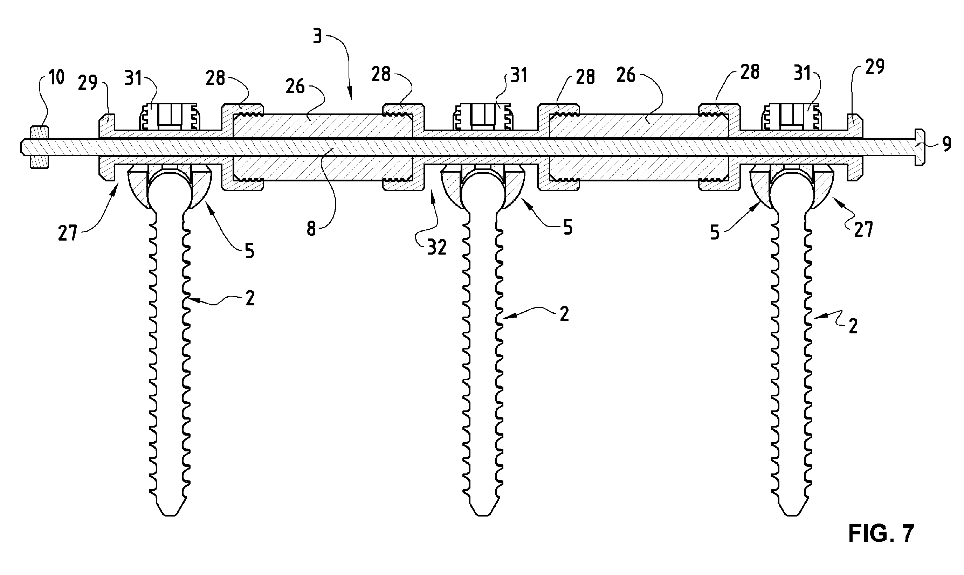

Fig. 7 zeigt ein gemäss dem Wirbelsäulenimplantat 1, wie es in den Fig. 5 und 6 dargestellt ist, entsprechendes Wirbelsäulenimplantat, wobei dieses drei Pedikelschrauben 2 umfasst. In die beiden äusseren Pedikelschrauben 2 ist jeweils ein hantelförmiges Element 27 eingesetzt und über die Spannschrauben 31 festgeklemmt, in die mittlere Pedikelschraube 2 ist ein hantelförmiges Element 32 eingesetzt, welches beidseitig jeweils eine Kappe 28 aufweist, mit welchen dieses hantelförmige Element 32 beidseitig mit den elastischen Zwischenteilen 26 formschlüssig verbunden ist.

Die Funktionsweise dieses in Fig. 7 dargestellten Wirbelsäulenimplantates 1 entspricht im Wesentlichen demjenigen, wie es in Fig. 3 dargestellt ist, beim Beugen der Wirbelsäule nach hinten werden die elastischen Zwischenteile 26 zusammengedrückt, wodurch eine dämpfende Abstützung der Wirbelsäule erreicht wird, beim Beugen der Wirbelsäule nach vorne werden die elastischen Zwischenteile 26 gedehnt, wobei die maximale Biegebewegung der Wirbelkörper, in welche die Pedikelschrauben 2 eingesetzt sind, durch die Anschlagelemente 9 und 10 des Längselementes 8 begrenzt wird.

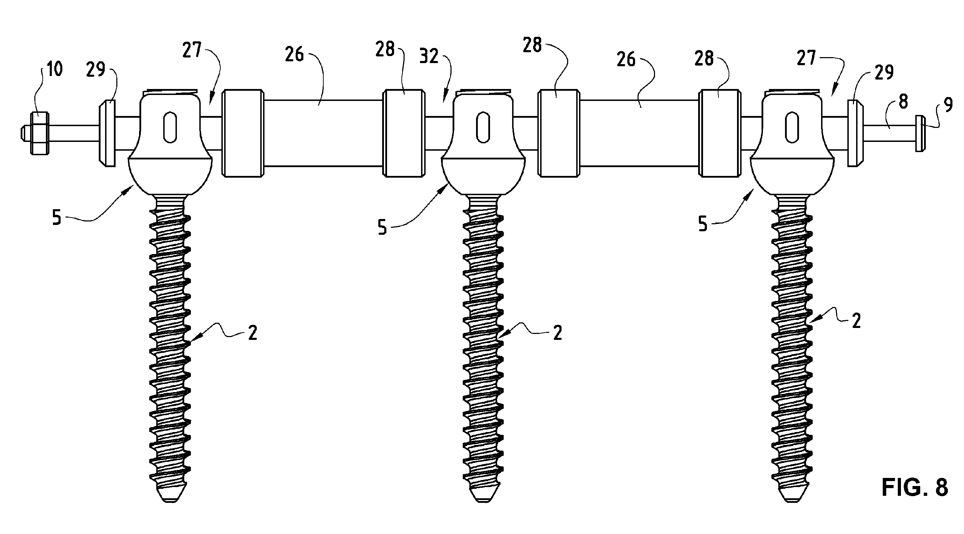

Fig. 8 zeigt eine Ansicht auf das Wirbelsäulenimplantat 1 gemäss der Fig. 7, einander entsprechende Elemente sind mit denselben Bezugszeichen versehen.

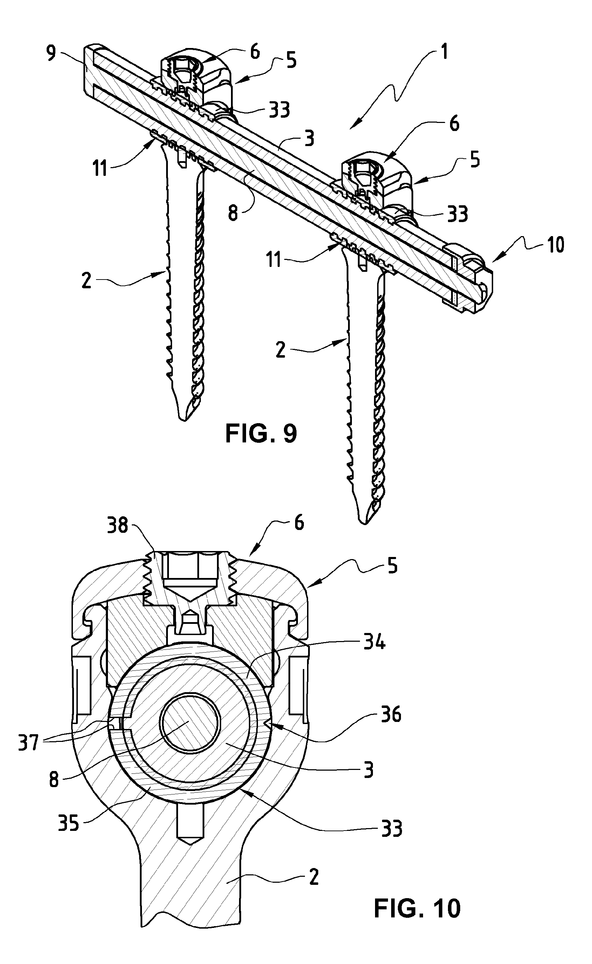

Fig. 9 zeigt ein Wirbelsäulenimplantat 1, das dem Wirbelsäulenimplantat entspricht, das in den Fig. 1 und 2 dargestellt ist, der einzige Unterschied besteht darin, dass das aus elastischem Material bestehende stabförmige Verbindungselement 3 nicht über die gesamte Länge mit einer strukturierten Oberfläche versehen ist, sondern dass sich diese strukturierte Oberfläche 11 auf die Bereiche beschränkt, die im Einflussbereich der Pedikelschrauben 2 liegen. Hierbei wird auf das stabförmige Verbindungselement 3 jeweils im Bereich der Pedikelschrauben 2 eine Hülse 33 aufgesetzt, über die strukturierten Oberflächen der Hülsen 33 und des stabförmigen Verbindungselementes 3 ergibt sich auch hier eine im wesentlichen formschlüssige Verbindung. Diese Lösung ist aus der europäischen Patentanmeldung mit der Anmeldenummer 08156089.8 bekannt. Zwischen Hülse 33 und Kopfteil 5 der Pedikelschraube wird eine Klemmverbindung erhalten. Mit dem Einsatz dieser Hülsen 33 wird die Montage des stabförmigen Verbindungselementes 3 vereinfacht, die Oberfläche des stabförmigen Verbindungselementes im Bereich der Hülse kann hierbei glatt oder strukturiert sein. Die Strukturierung der Oberfläche kann hierbei auf den Bereich der Hülse beschränkt werden, sie kann sich aber auch über die gesamte Länge des stabförmigen Verbindungselementes erstrecken. Durch die Verwendung einer Hülse 33 wird erreicht, dass die Druckspitzen der Einspannkräfte im Kunststoff minimiert werden können. Zudem wird die Längsbohrung im stabförmigen Verbindungselement nicht übermässig verformt, die gewünschte Funktionsweise ist gewährleistet. Zwischen dem Kopfteil der Pedikelschraube und der Hülse wird eine reine Pressverbindung erhalten, was zu einer sehr einfachen Montage führt. Auch hier können bei normalen Durchmessern der Hülse standardmässige Pedikelschrauben eingesetzt werden.

In der Schnittdarstellung gemäss Fig. 10 ist der Kopfteil 5 der Pedikelschraube 2 ersichtlich, in welche das stabförmige Verbindungselement 3 mit dem eingeführten Längselement 8 eingesetzt ist. Um das stabförmige Verbindungselement 3 ist die Hülse 33 gelegt, wobei diese aus zwei Hälften 34 und 35 besteht, die auf der einen Längsseite über ein Gelenk 36 miteinander schwenkbar verbunden sind. Auf der gegenüberliegenden Längsseite weisen die jeweiligen Hälften 34 und 35 der Hülse 33 jeweils eine Anschlagfläche 37 auf, die im voll gespannten Zustand der Spannschraube 38 der Verschlusseinrichtung 6 aneinander stossen. Dadurch ist gewährleistet, dass das elastische stabförmige Verbindungselement 3 bei vollständig eingespannter Position nicht zu stark zusammengepresst wird.

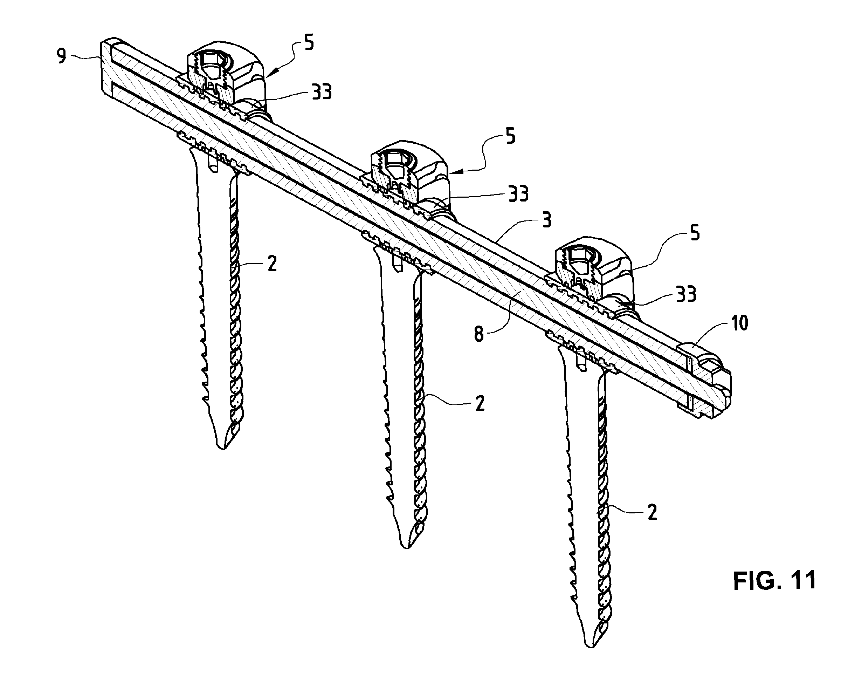

Fig. 11 zeigt ein Wirbelsäulenimplantat 1, das demjenigen entspricht, das in Fig. 3 dargestellt ist, wobei auch hier im Verbindungsbereich Pedikelschraube 2 - stabförmiges Verbindungselement 3 jeweils eine Hülse 33, die zu Fig. 10 im Detail beschrieben worden ist, eingesetzt ist. Die Funktionsweise dieses Wirbelsäulenimplantats entspricht derjenigen des in Fig. 2 dargestellten und dort beschriebenen Wirbelsäulenimplantats.

Die vorgängig beschriebenen Wirbelsäulenimplantate umfassen alle ein stabförmiges Verbindungselement, das mit einer durchgehenden Längsbohrung 7 ausgestattet ist, in welche das Längselement 8 eingesetzt ist. Alle stabförmigen Verbindungselemente 3 können auf dem jeweiligen Längselement 8 gleiten. Für das Längselement 8 kann jeweils ein geeignetes Material ausgesucht werden, abhängig davon, welchen Beanspruchungen dieses Längselement 8 ausgesetzt ist. Das Material kann beispielsweise eine Titanlegierung sein, insbesondere dann, wenn dieses Längselement 8 sowohl auf Zug als auch auf Druck beansprucht wird, und wenn entsprechende translatorische Kräfte aufgenommen werden müssen. Dieses Längselement 8 kann aber auch aus einem geeigneten Kunststoff bestehen, insbesondere dann, wenn nur geringe translatorische Bewegungen aufgenommen werden müssen, wobei dieses Material zumindest eine relativ grosse Zugfestigkeit aufweisen soll. Ferner kann dieses Längselement auch aus einem Kabel oder Seil aus einem metallischen Werkstoff oder Kunststoff (z.B. Polyester) gebildet sein, insbesondere dann, wenn dieses Längselement nur auf Zug beansprucht werden soll. Diese erfindungsgemässen Wirbelsäulenimplantate ermöglichen, diese jeweils bedarfsgerecht an den jeweiligen Fall anzupassen, wodurch eine optimale Abstützung und Stabilisierung der jeweiligen Wirbelsäule erreichbar ist.

## Patentansprüche

1. Wirbelsäulenimplantat, umfassend Pedikelschrauben (2), die jeweils mit einem Kopfteil (5) und einem Einschraubteil (4) ausgestattet sind, welche mit den Einschraubteilen (4) in die Wirbelkörper einschraubbar sind, mindestens ein stabförmiges Verbindungselement (3), das mindestens Teilbereiche aufweist, die aus einem elastischen Material gebildet sind, welche gegebenenfalls mit aus starrem Material bestehenden Zwischenstücken verbunden sind, mit welchem stabförmigem Verbindungselement (3) die Pedikelschrauben (2) miteinander verbindbar sind, welche elastischen Bereiche mindestens teilweise formschlüssig mit den Kopfteilen (5) der Pedikelschrauben (2) beziehungsweise mindestens teilweise formschlüssig mit den Zwischenstücken verbindbar sind, **dadurch gekennzeichnet, dass** das stabförmige Verbindungselement (3) mit einer durchgehenden Längsbohrung (7) versehen ist, in welche ein im wesentlichen zug- und/oder druckstabiles Längselement (8) eingesetzt ist, das an beiden Endbereichen mit Anschlagelementen (9, 10) versehen ist, welche auf den stirnseitigen Endflächen des stabförmigen Verbindungselementes (3) abstützbar sind.

2. Wirbelsäulenimplantat nach Anspruch 1, **dadurch gekennzeichnet, dass** mindestens die Verbindungsstellen, mit welchen die elastischen Bereiche des stabförmigen Verbindungselementes (3) mit den Kopfteilen (5) der Pedikelschrauben (2) beziehungsweise mit den Zwischenstücken verbindbar sind, mit strukturierten Oberflächen (11) ausgestattet sind, welche mit entsprechend strukturierten Oberflächen der zu verbindenden Teile zusammenwirken und eine formschlüssige Verbindung ergeben.

3. Wirbelsäulenimplantat nach Anspruch 2, **dadurch gekennzeichnet, dass** die strukturierten Oberflächen (11) aus Rippen und Rillen (12, 13; 14,15) bestehen, die jeweils ineinander greifen.

4. Wirbelsäulenimplantat nach Anspruch 3, **dadurch gekennzeichnet, dass** die Rippen und Rillen (12, 13; 14, 15) schraubenlinienförmig ausgebildet sind.

5. Wirbelsäulenimplantat nach einem der Ansprüche 1 bis 4, **dadurch gekennzeichnet, dass** mindestens eines der beiden Anschlagelemente (9, 10) des Längselementes (8) verstellbar ausgebildet ist.

6. Wirbelsäulenimplantat nach Anspruch 5, **dadurch gekennzeichnet, dass** das verstellbare Anschlagelement (10) mit einem Innengewinde (17) versehen ist, welches auf den mit einem entsprechenden Gewinde (18) versehenen Endbereich des Längselementes (8) aufschraubbar und sicherbar ist.

7. Wirbelsäulenimplantat nach Anspruch 5 oder 6, **dadurch gekennzeichnet, dass** das verstellbare Anschlagelement (10) mit einer Kappe versehen ist, welche den Endbereich des stabförmigen Verbindungselementes (3) übergreift.

8. Wirbelsäulenimplantat nach Anspruch 7, **dadurch gekennzeichnet, dass** die Kappen (19, 22) der Anschlagelemente (9, 10) mit den aus elastischem Material bestehenden jeweiligen Endbereichen des stabförmigen Verbindungselementes (3) im Wesentlichen formschlüssig verbunden sind.

9. Wirbelsäulenimplantat nach Anspruch 8, **dadurch gekennzeichnet, dass** mindestens eine der beiden Kappen (19, 22) zwischen zwei Stellelementen (24, 25) verschiebbar auf dem Längselement (8) gehalten ist.

10. Wirbelsäulenimplantat nach einem der Ansprüche 1 bis 9, **dadurch gekennzeichnet, dass** auf die elastischen Bereiche des stabförmigen Verbindungselementes (3), wenn diese in die Kopfteile (5) der Pedikelschrauben (2) einzusetzen sind, Hülsen (33) aufgesetzt sind, welche den jeweiligen elastischen Bereich des stabförmigen Verbindungselementes (3) umschliessen.

11. Wirbelsäulenimplantat nach Anspruch 10, **dadurch gekennzeichnet, dass** die Hülsen (33) aus zwei Hälften (34, 35) gebildet sind, die auf der einen Längsseite gelenkig miteinander verbunden sind, während die gegenüberliegenden Längsseiten als Anschlagflächen (37) dienen.

12. Wirbelsäulenimplantat nach Anspruch 10 oder 11, **dadurch gekennzeichnet, dass** die Innenflächen der Hülsen (33) im Wesentlichen eine strukturierte Oberfläche aufweisen, welche der strukturierten Oberfläche (11) des stabförmigen Verbindungselementes (3) entspricht.
